# EUROPÄISCHE PATENTSCHRIFT

(11) **EP 3 352 803 B1**
(45) Veröffentlichungstag und Bekanntmachung des Hinweises auf die Patenterteilung: **04.11.2026**
(21) Anmeldenummer: 16766282.4
(22) Anmeldetag: 14.09.2016
(51) Int. Cl.: A61L 2/18

(54) **VERFAHREN ZUR ÖFFENTLICHEN SIGNALISIERUNG EINES DESINFEKTIONSZUSTANDES VON PERSONEN**
METHOD FOR PUBLICALLY SIGNALLING A DISINFECTED STATE OF PERSONS
PROCÉDÉ DE SIGNALISATION PUBLIQUE D'UN ÉTAT DE DÉSINFECTION DE PERSONNES

(30) Priorität: 21.09.2015 DE 102015218040
(43) Veröffentlichungstag der Anmeldung: 01.08.2018
(73) Patentinhaber: Heyfair GmbH, 07745 Jena (DE)
(72) Erfinder: Hellmundt, Robert, 99510 Apolda (DE)
(74) Vertreter: Meissner Bolte Partnerschaft mbB
(86) Internationale Anmeldenummer: PCT/EP2016/071708
(87) Internationale Veröffentlichungsnummer: WO 2017/050619

(56) Entgegenhaltungen:
- EP-A1- 1 184 071
- EP-A2- 1 163 913
- WO-A1-00/22260
- WO-A1-2008/019202
- WO-A1-2012/042243
- WO-A1-90/08558
- WO-A1-95/05998
- WO-A2-2007/090387
- WO-A2-2008/112073
- WO-A2-2013/030597
- CN-U- 201 537 558
- CN-U- 205 138 996
- US-A- 5 152 431
- US-A- 6 139 821
- US-A1- 2005 090 414
- US-A1- 2011 316 703
- US-A1- 2015 216 985
- US-A1- 2016 296 077
- DATABASE WPI Week 201060, Derwent World Patents Index; AN 2010-L08525, XP002768552

## Beschreibung

Die Erfindung betrifft ein nicht-therapeutisches und nicht-diagnostisches Verfahren zur öffentlichen Signalisierung eines Desinfektionszustandes von Personen.

Aus dem Stand der Technik sind Desinfektionsmittel und Verfahren zur Verwendung von Desinfektionsmitteln bekannt, bei denen auf Markierungsstoffe zurückgegriffen wird. So beschreibt beispielsweise die US-amerikanische Patentschrift US 7,053,029 B2 eine Seife, deren Verwendung signalisiert wird. Die Seife enthält einen Indikator, der nach einer bestimmten Zeit einen Farbumschlag oder eine Änderung der Viskosität durchläuft, wobei dem Benutzer hierdurch signalisiert wird, dass ein mit der Seife ausgeführter Reinigungsvorgang, beispielsweise das Waschen der Hände, mit einem gewissen zeitlichen Minimum erfolgen muss. Die Änderung des Indikators signalisiert dem Benutzer, dass das notwendige zeitliche Minimum des Waschvorgangs erreicht worden ist.

Die US 6,139,821 offenbart Materialien und Verfahren unter Verwendung eines temporären visuellen Indikators. Die Druckschrift lehrt ein Sonnenschutzmittel mit einem Indikator, der beim Auftragen auf die Haut sichtbar ist, aber kurz nach dem Auftragen in einen unsichtbaren Zustand übergeht. Der Indikator verbleibt jedoch auf der Haut und kann bedarfsweise wieder aktiviert werden, damit überprüft werden kann, ob die Sonnenschutzcreme noch in einer hinreichenden Menge auf der Haut vorhanden ist. Während der normalen Anwendung ist der Indikator nicht sichtbar, damit eine nachteilige Wechselwirkung mit der Sonnenschutzwirkung vermieden und die Bräunung der Haut nicht durch eine Schattenbildung beeinträchtigt wird.

Weiterhin sind aus verschiedenen Druckschriften, beispielsweise der WO 2008/112073 oder der WO 2008/060355 A2 Seifen und Desinfektionsmittel bekannt, bei denen auf der Hand oder auf zu desinfizierenden Oberflächen und Instrumenten Einwirkzeiten angezeigt werden, wobei beispielsweise signalisiert werden soll, ob das aufgebrachte Desinfektionsmittel sich nicht mehr in einem entflammbaren Zustand befindet.

Die genannten Desinfektionsmittel zeigen somit einen richtigen Gebrauch an und signalisieren den Personen, die diese unmittelbar verwenden, beispielsweise einem Operateur, einem behandelnden Arzt oder dem Klinikpersonal, welches mit der Desinfizierung von medizinischen Geräten beauftragt ist, dass sich der zu desinfizierende Bereich in einem ordnungsgemäßen Zustand befindet.

In Bereichen mit hohem Publikumsverkehr, insbesondere Kliniken, Krankenstationen, aber auch in öffentlichen Gebäuden, Behörden oder anderen Orten, bei denen entweder auf einer erhöhte Sauberkeit geachtet werden muss oder bei denen die Gefahr übertragbarer Krankheiten infolge einer hoher Dichte an Menschen besonders hoch und daher durch entsprechende Sauberkeits- und Desinfizierungsmaßnahmen gesichert werden muss, können die genannten Desinfektionsmittel jedoch keine Anwendung finden. Dass ein Desinfektionsmittel richtig angewendet worden ist, dass also beispielsweise der Desinfektionsvorgang lange genug gedauert hat und nun beendet werden kann, wird nur dem unmittelbaren Anwender gegenüber angezeigt.

Wichtig ist aber im Gegensatz dazu, dass jeder innerhalb des Publikums in dem medizinischen Bereich, dem öffentlichen Gebäude oder der Behörde eine hinreichende Sicherheit darüber erlangen kann, dass sich seine ihn umgebenden Mitmenschen Desinfektionsmaßnahmen unterzogen haben. In einem solchen Falle ist es nur von sekundärer Bedeutung, ob sich jemand lang genug die Hände gewaschen oder mit Desinfektionsmittel eingerieben hat. Viel wichtiger ist es, zu wissen ob sich jemand überhaupt desinfiziert hat, um genau und schnell erkennen zu können, ob deswegen eine Person in einen bestimmten Bereich Zutritt bekommen darf oder nicht. Die bekannten Desinfektionsmittel sind hierzu nicht verwendbar.

Es ergibt sich daher die Aufgabe, ein Verfahren anzugeben, mit dem eine derartige öffentliche und für jedermann eindeutig sichtbare Kommunikation des Desinfektionszustandes jeder einzelnen Person ohne Umstände möglich ist. Sofern hierzu besondere Vorrichtungen notwendig sind, sollen diese leicht handhabbar sein, sicher funktionieren und mit einem minimalen Aufwand installierbar sein.

Der Desinfektionszustand der Person soll über einen hinreichend langen Zeitraum verfolgbar sein. Und zwar auch dann, wenn der Desinfektionsvorgang als solcher schon einige Zeit zurückliegt. Es geht hier vor allem um eine verlässliche Signalisierung darüber, ab wann der desinfizierte Zustand nicht mehr vorliegt oder von Beginn an nicht vorgelegen hat.

Schließlich besteht außerdem die Aufgabe, ein Kommunikationsverfahren anzugeben, mit dem insgesamt auf eine leichte Art und Weise der Desinfektionszustand öffentlich signalisiert werden kann.

Die genannten Aufgaben werden mit einem nicht-therapeutischen und nichtdiagnostischen Verfahren zur öffentlichen Signalisierung eines Desinfektionszustandes mit den Merkmalen des Anspruchs 1 gelöst.

Die im Folgenden genannten Ausführungsformen des Desinfektionsmittelspenders, des Desinfektionsmittels sind nicht erfindungsgemäß und können im Rahmen des Signalisierungsverfahrens nach Anspruch 1 zur Anwendung gelangen.

Ein hier nicht erfindungswesentlicher Desinfektionsmittelspender zur Bevorratung und Entnahme eines Mittels zur Oberflächendesinfektion umfasst einen ersten Vorratsbehälter mit einem darin enthaltenen Desinfektionsmittel, einen zweiten Vorratsbehälter mit einem darin enthaltenen Markierungsstoff, wobei der Markierungsstoff innerhalb des zweiten Vorratsbehälters inaktiv, und einen Mischabschnitt, in welchem der Markierungsstoff bei einer Betätigung des Desinfektionsmittelspenders dem Desinfektionsmittel beigemischt wird.

Der Markierungsstoff liegt dann nach dem Entnahmevorgang aus dem Desinfektionsmittelspender in einem instabilen Zustand vor.

Grundidee des Desinfektionsmittelspenders ist es, mit dieser Vorrichtung ein Desinfektionsmittel zu markieren. Der Markierungsstoff wird durch den Desinfektionsmittelspender dem Desinfektionsmittel beigemischt. Er befindet sich nach dem Desinfektionsvorgang somit zusammen mit dem Desinfektionsmittel auf dem desinfizierten Areal, beispielsweise auf der desinfizierten Hand. Der Markierungsstoff ist innerhalb des Desinfektionsmittelspenders inaktiv und kann praktisch beliebig lange bevorratet werden. Nach der Beimischung zum Desinfektionsmittel, spätestens aber nach dessen Entnahme und nach der Auftragung auf der desinfizierten Oberfläche ist er aktiviert und zerfällt mit der Zeit, während er hinreichend lange sichtbar bleibt. Die Idee ist es, den Desinfektionsmittelspender gleichzeitig zu einer Vorrichtung zu gestalten, mit der Personen markiert werden. Und zwar so, dass die Desinfektion zwingend mit deren Markierung verbunden werden kann, wobei aber die Markierung nach einer bestimmten Zeit wieder verschwindet.

Bei einer weiteren Ausführungsform enthält der Desinfektionsmittelspender einen Aktivierungsabschnitt, in welchem der Markierungsstoff von dem inaktiven Zustand in den aktivierten Zustand überführbar ist. Dieser Abschnitt dient dazu, den in dem Desinfektionsmittelspender enthaltenen Markierungsstoff so zu beeinflussen, dass dieser seiner Markierungsfunktion genügen kann, aber sich im Vorrat des Desinfektionsmittelspenders selbst nicht zersetzt.

Der Aktivierungsabschnitt kann auf verschiedene Weise ausgebildet sein:
Der Aktivierungsabschnitt kann ein Mittel für einen Wärmeeintrag sein, wobei durch den Wärmeeintrag das Überführen des Markierungsstoffs in den aktivierten Zustand bewirkbar ist.

Der Aktivierungsabschnitt kann ein Mittel zum Eintragen elektromagnetischer Strahlung enthalten, wobei durch die elektromagnetische Strahlung das Überführen des Markierungsstoffs in den aktivierten Zustand bewirkbar ist. Dies kann beispielsweise ultraviolette Strahlung sein, die die Molekülbildungen des Markierungsstoffs aufbricht, wobei die Reaktionsprodukte zum einen instabil sind und zum anderen aber entsprechend eine Farbbildung erzeugen und daher zum Markieren geeignet sind.

Der Aktivierungsabschnitt kann ein rheologisch wirksames Mittel zur Beeinflussung des Fließverhaltens und/oder der inneren Reibung von Flüssigkeiten sein, wobei durch das rheologische Mittel ein Freisetzen mikroverkapselter Markierungsstoffmengen in das Volumen des Desinfektionsmittels bewirkbar ist. Hierbei ist es möglich, die innere Reibung einer strömenden Flüssigkeit so auszunutzen, dass der Markierungsstoff aus einer ihn stabilisierenden verkapselten Form freigesetzt wird und dadurch aktiviert wird, während er aber gleichzeitig die zeitlich begrenzte Markierungsfunktion erfüllen kann.

Bei einer Ausführungsform ist der Desinfektionsmittelspender in Form eines doppelwandigen Behältnisses, insbesondere einer doppelwandigen Tube, ausgebildet, wobei der Vorratsbehälter für den Markierungsstoff durch den Innenhohlraum oder den Doppelwandhohlraum des Behältnisses gebildet ist. Eine solche Ausführungsform eignet sich besonders für tragbare Desinfektionsmittelspender.

Ein Desinfektionsmittel zur Oberflächendesinfektion von Gegenständen und Körperteilen gemäß der Erfindung besteht einer desinfizierenden Grundkomponente und einem der Grundkomponenten wahlweise zumischbaren Markierungsstoff. Der Markierungsstoff ist vor dem Zumischen zur desinfizierenden Grundkomponente stabil und markiert nach dem Zumischen und Auftragen des Desinfektionsmittels auf eine Oberfläche diese Oberfläche, wobei die markierende Wirkung sich bei nachlassendem Desinfektionszustand verflüchtigt.

Das Desinfektionsmittel desinfiziert und markiert gleichermaßen. Die desinfizierende Grundkomponente bewirkt die Desinfektion als solche, der Markierungsstoff wird dabei mit aufgetragen und zeigt die Desinfektion des entsprechenden Körperteils oder des Gegenstandes an. Die markierende Wirkung des Markierungsstoffs verflüchtigt sich genau dann, wenn die desinfizierende Wirkung der desinfizierenden Grundkomponente nachlässt.

Bei einer ersten Ausführungsform ist der Markierungsstoff ein visuell sichtbarer Farbstoff. Der Markierungsstoff kann ein durch Energieeintrag fluoreszierender Farbstoff sein. Der Markierungsstoff kann ein im nicht sichtbaren Bereich nachweisbarer Farbstoff sein. Möglich ist also neben der reinen visuell sichtbaren Markierung auch eine Markierungsform, die im nicht sichtbaren Bereich, beispielsweise durch geeignete Kamerasysteme oder durch gewisse Anregungen, beispielsweise durch Bestrahlung mit so genanntem Schwarzlicht, sichtbar wird. Dies hat den Vorteil, dass hier objektivere und dokumentierbare Erfassungen und Kontrollen von Personen vorgenommen werden können, die durch einfachere Betrachtungen mit bloßem Auge und im individuellen Urteil der Beobachter so nicht möglich sind.

Bei einer Ausführungsform ist der Markierungsstoff durch die Wechselwirkung mit der desinfizierenden Grundkomponente oder durch eine externe Einwirkung von dem stabilen Zustand in den flüchtigen oder zersetzlichen Zustand überführbar. Genau dieser Fall tritt ein, wenn der Markierungsstoff zusammen mit der desinfizierenden Komponente verwendet wird. In diesem Fall markiert er die Person, die gleichzeitig desinfiziert ist. Der Markierungsstoff ist aber genau ab diesen Zeitpunkt zersetzlich, er verflüchtigt sich quasi mit der desinfizierenden Wirkung der desinfizierenden Komponente.

Der Markierungsstoff kann auch durch die Wechselwirkung mit der desinfizierenden Grundkomponente von dem stabilen Zustand in einen zweiten, metastabilen Zustand überführbar sein. Dabei geht der metastabile Zustand des Markierungsstoffes durch eine Wechselwirkung mit der Oberfläche des desinfizierten Gegenstandes und/oder einer umgebenden Atmosphäre in den flüchtigen oder zersetzlichen Zustand über. In einem solchen Fall wird der Markierungsstoff nicht im eigentlichen Sinne durch die desinfizierende Grundkomponente zersetzt. Jedoch ist die Anwesenheit der desinfizierenden Grundkomponente die Voraussetzung dafür, dass der Markierungsstoff beispielsweise durch die umgebende Luft oder durch andere Einwirkungen zersetzt werden kann.

In Verbindung damit kann die markierende Wirkung des Markierungsstoffs während des flüchtigen oder zersetzlichen Zustand des Markierungsstoffs auch erst auftreten und aktiviert werden. Das bedeutet, dass der Markierungsstoff erst dann wirklich markierend wirkt, d.h. beispielsweise als Einfärbung sichtbar ist, während er sich zusammen mit dem Desinfektionsmittel auf der desinfizierten Hand befindet. Befindet sich der Markierungsstoff dagegen ohne die desinfizierende Komponente auf der Hand, dann markiert er nicht und es kann keine Desinfektion vorgetäuscht werden. Eine derart nicht markierte Person wird daher auch nicht als desinfizierte Person wahrgenommen oder registriert.

Erfindungsgemäß werden bei dem Verfahren zur öffentlichen Signalisierung eines Desinfektionszustandes von Personen folgende Verfahrensschritte ausgeführt:
Es erfolgt ein Auftragen einer desinfizierenden Mischung bestehend aus einer desinfizierenden Komponente und einer Markierungskomponente auf das zu desinfizierende Hautareal an einer Desinfektionsstation und/oder aus einem tragbaren Desinfektionsmittelspender. Dadurch wird ein Einfärben des zu desinfizierenden Hautareals durch die Markierungskomponente während des Desinfektionsvorgangs bewirkt. Hierdurch kommt es zu einer sichtbaren Persistenz der Einfärbung auf dem desinfizierten Hautareal, wobei der desinfizierte Zustand des Hautareals allgemein wahrnehmbar signalisiert wird. Schließlich klingt die Persistenz der Einfärbung spätestens mit nachlassender Sterilität des Hautareals wieder ab.

Erfindungsgemäß wird das Abklingen der Markierkomponente durch das Verschwinden der Einfärbung infolge des Einwirkens der Umgebungsluft und/oder durch ein Ausbleichen infolge der Einwirkung von Umgebungslicht ausgelöst.

Bei einer Ausgestaltung des Verfahrens setzt die sichtbare Persistenz der Einfärbung auf dem desinfizierten Hautareal frühestens dann ein, wenn durch die desinfizierende Komponente die Sterilität des Hautareals bewirkt wird. Das bedeutet insbesondere, dass unmittelbar mit dem Desinfizieren zunächst keine Einfärbung erfolgt. Diese beginnt erst, wenn die desinfizierende Komponente tatsächlich ihre keimabtötende Wirkung entfaltet und bleibt aber dann solange bestehen, bis spätestens die Sterilität des Hautareals wieder abklingt.

Bei einer Ausgestaltung des Verfahrens wird die sichtbare Persistenz der Einfärbung auf dem Hautareal durch die abtötende Wirkung der desinfizierenden Komponente erzeugt, wobei dadurch entstehende biologische Abbauprodukte in der Markierkomponente eine sichtbare Farbreaktion erzeugen. Das bedeutet, dass solange dann, wenn die Stoffe, die durch die infolge des Desinfektionsvorgangs abgetöteten Keime freigesetzt werden, auch die Einfärbung verbleibt. Ist diese Freisetzung aber abgeschlossen, werden also keine Keime mehr abgetötet und ist daher auch das desinfizierte Areal nicht mehr steril, so wird dadurch die Einfärbung nicht mehr aufrechterhalten und der nicht sterile Zustand ist anhand der fehlenden Einfärbung wieder allgemein sichtbar.

Bei einer Ausführungsform wird die markierende Wirkung des Markierungsstoffs durch eine molekulare Schalterstruktur bewirkt, wobei die molekulare Schalterstruktur durch eine erste elektromagnetische Bestrahlung zu einem farblich wahrnehmbaren und/oder detektierbaren Zustand des Markierungsstoffs führt und durch eine zweite elektromagnetische Bestrahlung in einen farblich nicht wahrnehmbaren und/oder nicht detektierbaren Zustand des Markierungsstoffs zurücksetzbar ist.

Bei einer Ausführungsform des Verfahrens wird das desinfizierte und eingefärbte Hautareal durch eine Zugangskontrolleinrichtung registriert, wobei im Ergebnis des Registrierungsvorgangs die Zugangskontrolleinrichtung einen Personenzutritt gewährt oder sperrt.

Bei einer weiteren Ausführungsform des Verfahrens wird das desinfizierte und eingefärbte Hautareal durch eine Bilderfassungs- und Bilderkennungseinrichtung registriert, wobei im Ergebnis einer ausgeführten Bilderfassung und Bilderkennung eine Desinfektionsüberwachung sich in einem Bereich aufhaltender Personenmengen und/oder durch vorbestimmte Durchgangsbereiche strömender Personenmengen ausgeführt wird.

Bei einer weiteren Ausführungsform wird die sichtbare Persistenz der Einfärbung auf dem Hautareal durch das Einsetzen eines Verdunstungsvorgangs der desinfizierenden Komponente ausgelöst, wobei hierdurch in der Markierkomponente eine Farbreaktion erfolgt. In einem solchen Fall wird demnach angezeigt, wenn die desinfizierende Komponente verdunstet. Erfahrungsgemäß setzt genau dann deren desinfizierende Wirkung ein, die über die Einfärbung nunmehr sichtbar wird.

Erfindungsgemäß wird das Abklingen der der Markierkomponente durch das Verschwinden der Einfärbung infolge des Einwirkens der Umgebungsluft und/oder durch ein Ausbleichen infolge der Einwirkung von Umgebungslicht ausgelöst.

Bei einer weiteren Ausführungsform wird ein Abklingen der Persistenz der Markierkomponente durch ein Einwirken eines zweiten Desinfektionsmittels ausgelöst. Bei dieser Ausführungsform kann die Verwendung mehrerer verschiedener Desinfektionsmittel visuell nachgeprüft werden.

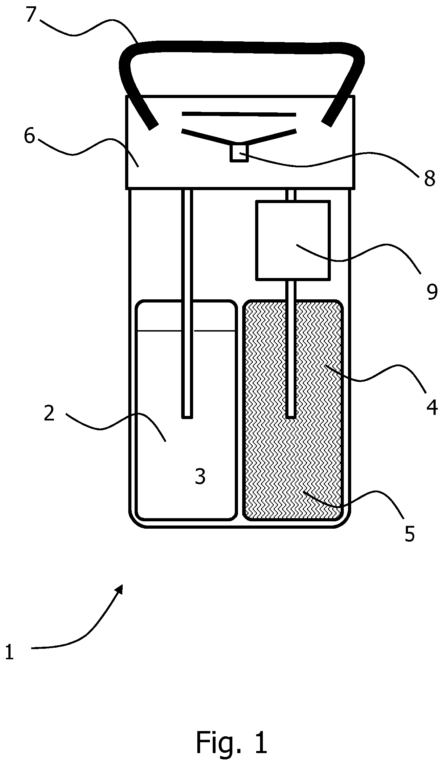

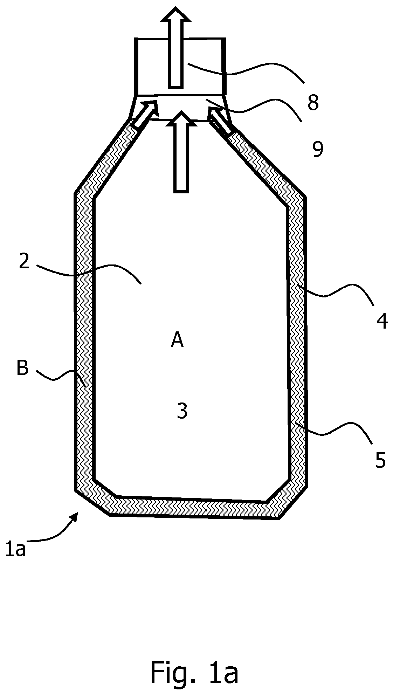

Der Desinfektionsmittelspender, das Desinfektionsmittel und das Verfahren zur öffentlichen Signalisierung eines Desinfektionszustandes von Personen soll nachfolgend anhand von beispielhaften Ausführungsformen näher erläutert werden. Zur Verdeutlichung dienen die Figuren 1 bis 3. Es werden für gleiche bzw. gleichwirkende Teile dieselben Bezugszeichen verwendet.

Es zeigt:
- Fig. 1: einen beispielhaften Desinfektionsmittelspender zur Verwendung mit einem markierenden Desinfektionsmittel und zur Verwendung im erfindungsgemäßen Signalisierungsverfahren,
- Fig. 1a: einen in Form eines doppelwandigen Behältnisses ausgebildeten Desinfektionsmittelspender,
- Fig. 2: eine Reihe unterschiedliche und möglicher Ausführungsformen für Desinfektionsmittelspender in Verbindung mit möglichen Ausführungsformen des markierenden Desinfektionsmittels,
- Fig. 3: einen beispielhaften Ablauf des erfindungsgemäßen Signalisierungsverfahrens mit einer Reihe beispielhafter Verfahrenskomponenten.

Fig. 1 zeigt einen beispielhaften Desinfektionsmittelspender 1. Der Desinfektionsmittelspender 1 enthält einen ersten Vorratsbehälter 2. In dem ersten Vorratsbehälter ist ein Desinfektionsmittel 3 enthalten. Das Desinfektionsmittel kann auf unterschiedlichen Wirkstoffen beruhen. Es können hier insbesondere sowohl alkoholbasierte als auch nicht alkoholbasierte Desinfektionsmittel verwendet werden.

Ein zweiter Vorratsbehälter 4 enthält einen Markierungsstoff 5. Der in diesem Beispiel gezeigte Desinfektionsmittelspender ist für eine stationäre Verwendung vorgesehen, bei dem die Vorrichtung an einem bestimmten Ort, beispielsweise in einem Eingangsbereich zu einem Gebäude, fest installiert ist. Möglich ist aber auch eine hier beispielhaft in Fig. 1a gezeigte mobile und tragbare Version, bei welcher beispielsweise in einer doppelwandigen Tube das Desinfektionsmittel im ersten Tubenvolumen und der Markierungsstoff im zweiten Tubenvolumen bevorratet sind. Ebenso können entsprechende Doppelkammer-Behältnisse, insbesondere tragbare Flaschen aus Kunststoff, als Desinfektionsmittelspender vorgesehen sein. Diese können beispielsweise in Eingangsbereichen im Publikum verteilt werden.

Bei der hier gezeigten stationären Ausführungsform des Desinfektionsmittelspenders ist eine Entnahmevorrichtung 6 vorgesehen, die im hier vorliegenden Beispiel mittels eines Bügels 7 betätigt werden kann. Durch diese Betätigung werden das Desinfektionsmittel 3 und der Markierungsstoff 5 aus ihren jeweiligen Vorratsbehältern herausgepumpt und vereinigen sich spätestens innerhalb einer Ausgabeöffnung 8 zu einem markierten Desinfektionsmittel, das beispielsweise auf eine darunter gehaltene Hand auftropft und dort verrieben werden kann, wobei die Hautoberfläche der Hand desinfiziert wird.

Innerhalb des Vorratsbehälters 4, bzw. in einem dazu entsprechenden Vorratsbehältnis bei einer mobilen oder tragbaren Version des Desinfektionsmittelspenders, befindet sich der Markierungsstoff in einem inaktiven Zustand. Er kann in diesem Behälter für eine grundsätzlich beliebig lange Zeit gelagert und transportiert werden. Beim Pumpvorgang während der Entnahme des Desinfizierungsmittels tritt der Markierungsstoff in einen Aktivierungsabschnitt 9 ein und verlässt diesen in einem aktivierten Zustand. Das abgegebene markierende Desinfektionsmittel stellt somit eine Mischung aus dem Desinfektionsmittel und dem aktivierten Markierungsstoff dar. Erst in diesem aktivierten Zustand entfaltet der Markierungsstoff seine markierende Wirkung und somit seine Signalfunktion.

Innerhalb des Vorratsbehälters 4 befindet sich der Markierungsstoff in dem inaktiven Zustand und kann nicht markierend eingesetzt werden. Er weist in diesem Zustand entweder keine markierende Wirkung auf und ist beispielsweise schlicht farblos oder er zeigt eine andere Farbe, die sich leicht von der eigentlichen Markierungsfarbe des Markierungsstoffes im aktivierten Zustand unterscheiden lässt.

Der Aktivierungsabschnitt 9 kann auf unterschiedliche Weise ausgebildet sein und sich auch an unterschiedlichen Positionen innerhalb des Desinfektionsmittelspenders befinden. Möglich ist hier insbesondere auch, dass der Aktivierungsabschnitt baulich mit der Ausgabeöffnung 8 zusammenfällt oder den Abschnitt umfasst, in welchem das Desinfektionsmittel und der zunächst inaktive Markierungsstoff das erste Mal zusammengeführt und dabei gemischt werden. In entsprechend vielfältiger Weise kann hierbei auch die Aktivierung des Markierungsstoffs im Aktivierungsabschnitt geschehen.

Die Aktivierung des Markierungsstoffs durch den Durchmischungsvorgang kann insbesondere bei mobilen und tragbaren Desinfektionsmittelspendern, d.h. bei doppelwandigen Tuben oder tragbaren Gebinden und Fläschchen angewendet werden. Diese doppelwandigen Vorrichtungen enthalten einen Innenhohlraum A und einen Doppelwandhohlraum B.

Bei der Ausführungsform aus Fig. 1a ist der Vorratsbehälter 4 des Markierungsstoffs im Doppelwandhohlraum B ausgebildet, während sich das Desinfektionsmittel im Innenhohlraum A befindet. Der im Doppelwandhohlraum enthaltene Markierungsstoff 5 tritt bei einem von außen ausgeübten Druck aus der Doppelwandung in den Aktivierungsabschnitt 9 aus und wird durch den dort stattfindenden Mischvorgang mit dem Desinfektionsmittel aktiviert. Das mit dem nunmehr aktivierten Markierungsstoff versetzte Desinfektionsmittel gelangt dann in die Ausgabeöffnung, die hier beispielsweise die Öffnung der doppelwandigen Tube ist und steht für die markierende Desinfektion bereit.

Ebenso ist auch eine andere, hier nicht gezeigte Ausführungsform eines stationären wie auch mobilen Desinfektionsmittelspenders möglich, bei der nur ein Vorratsbehälter vorhanden ist, der eine bereits fertige Mischung aus dem Desinfektionsmittel und dem Markierungsstoff enthält. Dabei enthält diese fertige Mischung den Markierungsstoff in inaktiver Form, wobei der Markierungsstoff spätestens auf der Haut oder der sonstigen zu desinfizierenden Fläche aktiviert wird und seine markierende Wirkung entfaltet.

Der Markierungsstoff kann auch als ein Stoff ausgebildet sein, dessen Moleküle als so genannte molekulare Schalter ausgebildet sind. Ein Beispiel hierfür sind Spiropyrane, die durch eine starke UV-Bestrahlung in farbiges Merocyanin umgewandelt werden und durch das Fehlen dieser Strahlung wieder in den farblosen Zustand zurückkehren. In einem solchen Fall ist der Aktivierungsabschnitt als ein UV-Strahler ausgebildet.

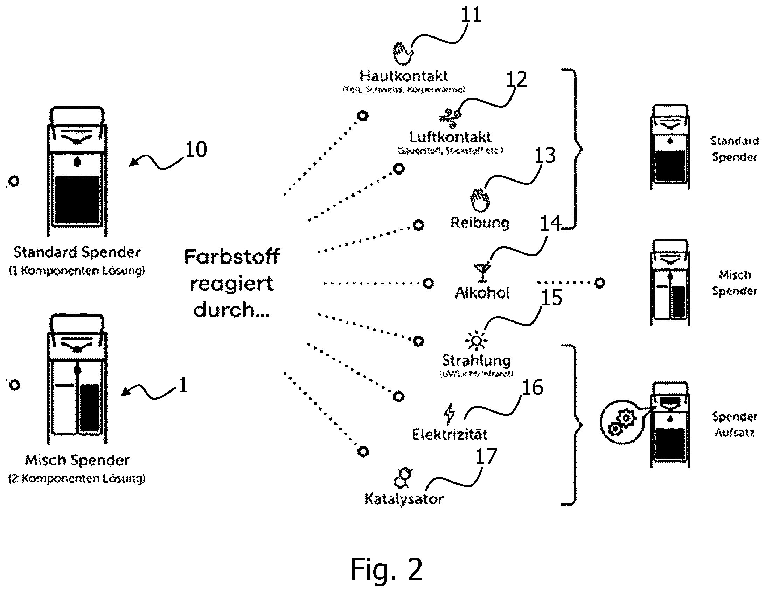

Figur 2 zeigt hierzu eine Reihe unterschiedlicher und möglicher Ausführungsformen für Desinfektionsmittelspender in Verbindung mit möglichen beispielhaften Ausführungsformen des markierenden Desinfektionsmittels.

Die Überblicksdarstellung in Fig. 2 zeigt links den in Fig. 1 gezeigten Desinfektionsmittelspender 1, der hier auch als Mischspender bezeichnet wird, und einen hier als Standardspender bezeichneten Desinfektionsmittelspender 10, bei dem das Desinfektionsmittel bereits mit dem Markierungsstoff gemischt ist.

In dem mittleren Bereich sind mögliche Aktivierungsvorgänge für den Markierungsstoff dargestellt. Die Aktivierung des Markierungsstoffs erfolgt beispielsweise durch einen Hautkontakt 11, einen Luftkontakt 12, durch Reibung 13, wobei dies insbesondere auch die innere Reibung bei strömenden Flüssigkeiten sein kann, durch ein Lösemittel 14, insbesondere durch das häufig bei Desinfektionsmitteln benutzte Lösemittel Ethanol, durch elektromagnetische Strahlung 15, insbesondere normales Tageslicht, durch Elektrizität 16, beispielsweise hier durch die piezoelektrische Aufladung eines Piezokristalls innerhalb der Entnahmevorrichtung 6, die bei der Entnahme erfolgt, oder durch einen entweder separaten oder im Desinfektionsmittel enthaltenen Katalysator 17, der die Aktivierung des Markierungsstoffs katalysiert.

Die Aktivierungsabschnitte im Desinfektionsmittelspender sind entsprechend unterschiedlich aufgebaut.

Bei einer Aktivierung des Markierungsstoffes durch Strahlung, insbesondere Licht, besteht der Aktivierungsabschnitt im einfachsten Fall aus einem Fenster, über das Umgebungslicht, beispielsweise normales Tageslicht oder das Licht von Raumbeleuchtungen auf den darin befindlichen und durch den Aktivierungsabschnitt beförderten Markierungsstoff einwirken kann. Bei einer anderen Variante einer Aktivierung des Markierungsstoffes durch Strahlung enthält der Aktivierungsabschnitt eine eigene Lichtquelle, die beispielsweise ultraviolettes oder infrarotes Licht auf den Markierungsstoff oder die Mischung aus dem Markierungsstoff und dem Desinfektionsmittel einstrahlt.

Bei einer Aktivierung des Markierungsstoffes mittels eines Katalysators besteht der Aktivierungsabschnitt aus einem Katalysatorfeld, über das der Markierungsstoff oder die Mischung aus dem Desinfektionsmittel und dem Aktivierungsstoff geleitet wird. Das Katalysatorfeld kann beispielsweise eine beschichtete Wandung sein, die Ausnehmungen zur Oberflächenvergrößerung aufweist. Bei einer anderen Variante besteht das Katalysatorfeld aus einer aus dem Katalysatormaterial bestehenden Füllung innerhalb einer Ampulle, durch die der Markierungsstoff oder die Mischung aus dem Markierungsstoff und dem Desinfektionsmittel hindurchgeleitet wird und dabei in einen intensiven Kontakt mit dem Katalysator tritt.

Bei einer Aktivierung des Markierungsstoffes auf mechanischem Wege liegt der Markierungsstoff zu Beginn in einer stabilisierenden Mikroverkapselung vor. Der Aktivierungsabschnitt weist hierzu mechanische Mittel auf, um die Mikroverkapselung aufzubrechen, wobei der Markierungsstoff freigesetzt wird und dabei in den instabilen und aktivierten Zustand übergeht. Diese mechanischen Mittel können auf unterschiedliche Weise ausgebildet sein. Bei einer ersten Variante handelt es sich hierbei um eine Anordnung aus mindestens zwei miteinander im Eingriff stehender Zahnräder oder miteinander im Kontakt stehender Walzen. Bei einer Betätigung der Entnahmevorrichtung am Desinfektionsmittelspender wird der mikroverkapselte Markierungsstoff durch die Zahnräder oder Walzen gepresst. Dabei werden die Mikroverkapselungen gequetscht und aufgebrochen, wobei die darin befindlichen Mengen des Markierungsstoffs freigesetzt werden. Die eigentliche Aktivierung des Markierungsstoffs erfolgt dann in dessen freigesetzten Zustand, beispielsweise durch dessen Wechselwirkung mit einem Lösemittel des Desinfektionsmittel oder der umgebenden Luft.

Möglich ist auch eine Ausführungsform des Aktivierungsabschnitt im Desinfektionsmittelspender, bei der der inaktive Markierungsstoff durch starken Druck komprimiert wird, oder bei dem dessen Teilchen zu Schwingungen angeregt werden, sodass hierdurch erst eine Reaktion ausgelöst wird, durch die der Markierungsstoff farbig wird und somit seine markierende Eigenschaft erst erhält.

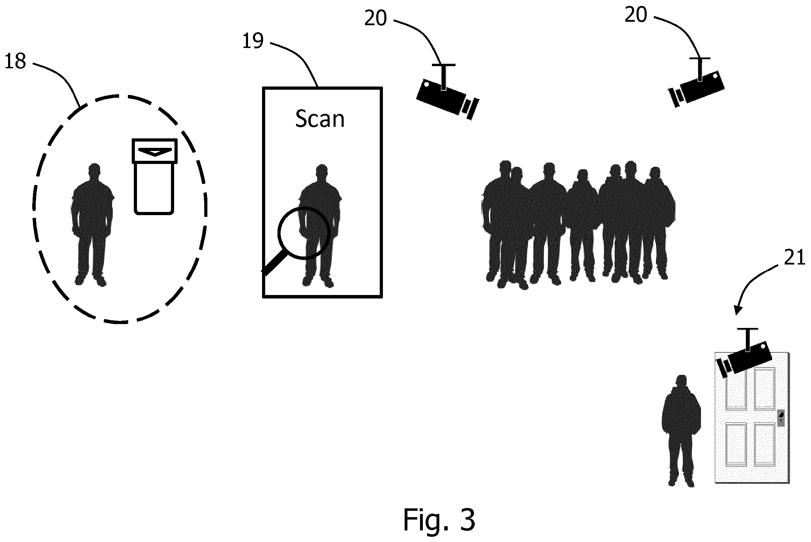

Fig. 3 zeigt einen beispielhaften Ablauf des Verfahrens zur Signalisierung des Desinfektionszustandes von Personen. Das Verfahren umfasst mehrere Einrichtungen und Komponenten, die an unterschiedlichen Orten innerhalb eines umgrenzten Gebietes, beispielsweise in einem Krankenhaus, einem Gebäudekomplex oder einem vergleichbaren Areal angeordnet sein. Darüber hinaus ist auch an eine Verwendung im Pflegebereich, insbesondere in Pflegeheimen, in Hotels und Gaststätten, in der Industrie sowie sonstigen Bereichen mit erhöhten Sauberkeits- und Hygieneanforderungen zu denken.

Ziel des Verfahrens ist es, auf die striktere Einhaltung von Hygienevorschriften hinzuwirken. Wie erwähnt wird dies dadurch erreicht, indem das eingesetzte Desinfektionsmittel eine zusätzliche signalisierende Eigenschaft erhält. Diese ist innerhalb einer vorbestimmten Zeitdauer nach dem Desinfektionsvorgang am Ort der Desinfektion, beispielsweise auf einer desinfizierten Hand, sichtbar. Hierdurch wird ein visuelles Feedbacksystem geschaffen, wodurch in der Wahrnehmung aller damit befassten Personen die Bedeutungsgleichheit "farbige Hände = sterile Hände" verankert wird. Jede Person kann für sich selbst aber auch durch die Beurteilung anderer Personen erkennen, wann deren Hände steril sind und wann nicht. Dadurch kann die Desinfektion kaum noch unbeabsichtigt vergessen werden. Zudem können nun auch andere Personen sehen, wann die Hände bei Personen in ihrer Umgebung steril sind oder nicht und somit bei einem Fehlverhalten korrigierend eingreifen. Aufgrund dieser Kontrolleigenschaften entsteht eine Verhaltensoptimierung, die insbesondere zu einer deutlichen Senkung der Infektionsrate führen kann.

Das Verfahren umfasst mindestens eine Desinfektionsstation 18, an dem die Desinfektion vorgenommen werden kann. Die Desinfektionsstation enthält hierzu insbesondere jeweils den oben erwähnten Desinfektionsmittelspender 1. Diese ist insbesondere nach einer der oben erwähnten Ausführungsformen ausgebildet.

Die Desinfektionsstationen sind über ein entsprechendes Gebiet verteilt und an zweckmäßigen Orten, beispielsweise im Eingangsbereich, einer Empfangshalle, vor den Zugängen besonderer Stationen und Bereiche oder an anderen zweckmäßigen Orten aufgestellt. Sie können sich insbesondere auch in Waschräumen oder Toiletten befinden.

Bei der Benutzung des Desinfektionsmittelspenders erfolgt eine eindeutig nachweisbare und visuell sichtbare Markierung des desinfizierten Körperteils der Person, indem die Person das Desinfektionsmittel einreibt und dabei die Haut einfärbt. Im Allgemeinen werden dies desinfizierte und dabei eingefärbte Hände sein. Diese visuelle Einfärbung signalisiert zum einen allen umgebenden Anwesenden, dass die Hände des Betreffenden sich in seinem desinfizierten Zustand befinden. Sie sind daher in einfacher Weise in der Lage, zwischen desinfizierten und nicht desinfizierten Personen zu unterscheiden und daraufhin ihr Verhalten einzurichten oder weitere Maßnahmen zu ergreifen.

Anstelle der stationären Desinfektionsstationen können auch tragbare Desinfektionsmittelspender vorgesehen sein. Diese können beispielsweise in Form von Tuben, tragbaren Spendern, Flaschen, Dosen usw. ausgebildet sein und am Mann getragen werden. Möglich ist in diesem Zusammenhang ein im Eingangsbereich vorhandenes Magazin oder ein Ausgabeautomat, in dem jeweils ein bestimmter Vorrat an tragbaren Desinfektionsmittelspendern vorhanden ist und an denen die eintretenden Personen die entsprechenden einzelnen Gebinde, Tuben, Flaschen und dergleichen Artikel entnehmen, wobei sie diese öffnen und sich durch das Auftragen des Inhalts beispielsweise auf die Hände desinfizieren.

Von besonderer Bedeutung ist diese Funktion in Kopplung mit einem bilderkennenden System zur Zugangskontrolle bzw. zur Überwachung von Menschengruppen. Das Verfahren umfasst hierzu die Verwendung eines Zugangskontrollsystems 19, beispielsweise in Form einer Zutrittsschleuse, die jede Person durchlaufen muss, um einen bestimmten Bereich zu betreten. Die Zugangskontrolle kann entweder in der Art erfolgen, indem die Person ihre desinfizierten Hände unter eine Scaneinrichtung halten muss, die bei dem Scanvorgang die Farbe die Hauteinfärbung erfasst und auswertet. Ermittelt beispielsweise die Detektion der Hand beispielsweise eine blaue Hautfärbung, so wird dies vom Scanner erkannt und die Person kann das Zugangskontrollsystem passieren. Weist dagegen die Hand einen typischen Hautfarbton oder eine andere Signalfarbe auf, so wird der Person das Passieren des Zugangskontrollsystems z.B. durch eine Schranke verwehrt.

Die Zugangskontrolle kann auch in einer weniger restriktiveren Weise erfolgen. In einem solchen Fall können Bilderfassungsvorrichtungen in der typischen Höhe angebraucht sein, in welcher sich die Hände vorüberlaufender Personen befinden. Hier wird ein Scanvorgang im Vorübergehen ausgeführt. Hierbei werden die Personen beim Passieren der Kontrolleinrichtung gegebenenfalls darauf hingewiesen werden, sich die Hände zu desinfizieren und sich deshalb zur nächstgelegenen Desinfektionsstation zu begeben, eine Desinfektionspackung zu entnehmen oder sich aus ihren mitgeführten Desinfektionspackungen zu desinfizieren. Eine derartige Einrichtung kann insbesondere in den Ausgängen sanitärer Anlagen angeordnet sein, wobei hier Personen durch ein entsprechendes Signal aufgefordert werden, sich zu desinfizieren.

Derartige Bilderfassungsvorrichtungen können auch praktisch an beliebigen Stellen vorhanden sein, die daran vorüberlaufenden Personen erfassen und mittels akustischer, optischer oder sprachgenerierter Signale zum Desinfizieren bzw. zum Auffrischen der Desinfektion auffordern.

Einen weiteren Verfahrensaspekt stellt die Überwachung und Kontrolle größerer Personengruppen dar, die sich innerhalb eines Bereiches aufhalten. Hierzu sind im überwachten Bereich mehrere Kameras 20 angeordnet, die mit einer hier nicht dargestellten zentralen Auswerteeinheit verbunden sind. Die Auswerteeinheit enthält Mittel zur Bilderkennung und Bildverarbeitung, insbesondere Mittel zur Registrierung von Farbtönen, die auf die Farbe des Markierungsstoffes eingerichtet sind. Auf diese Weise ist zum einen eine ungefähre statistische Erfassung darüber möglich, wie hoch der Anteil der desinfizierten Personen im überwachten Bereich ist und wie sich der Anteil der desinfizierten Personen räumlich über einen bestimmten Bereich verteilt. Dies kann die Grundlage dafür bieten, entsprechende Maßnahmen einzuleiten, um den Desinfektionszustand in den entsprechenden Bereichen zu verbessern.

Die Anwendung des Desinfektionsmittels gestaltet sich beispielsweise wie folgt:
Das Desinfektionsmittel ist innerhalb einer gewissen Mindestzeitdauer sichtbar. Diese Mindestzeitdauer kann beispielsweise etwa 1 Minute betragen. Eine obere Grenze dieses zeitlichen Intervalls kann grundsätzlich beliebig sein, die Sichtbarkeit des Desinfektionsmittels sollte jedoch spätestens dann wieder abklingen, sobald die desinfizierende Wirkung nachlässt.

Nach der Trocknung des Desinfektionsmittels auf den Händen, die in der Regel nicht mehr als 30 Sekunden dauert, bleibt der Markierungsstoff auf der Haut sichtbar und färbt nicht ab. Auf diese Weise entfaltet sich dessen Kontroll- und Signalwirkung. Gleichzeitig wird durch das Verschwinden der Färbung nach einer bestimmten Zeit vermittelt, dass die Hände des Anwenders nach dieser Zeitspanne höchstwahrscheinlich wieder kontaminiert sind. Es wird hierdurch signalisiert, wann es angebracht ist, die Hände erneut zu desinfizieren. Eine solche Vorgehensweise kann verfolgt werden, wenn an die Stärke der Keimfreiheit nur durchschnittliche Anforderungen im Sinne einer erhöhten Sauberkeit gestellt werden.

Als weitere Möglichkeit bietet es sich an, dass die Einfärbung der Haut nur so lange auftritt, bis das Desinfektionsmittel getrocknet ist und alle Keime mit hoher Sicherheit abgetötet wurden. So wird dem Anwender auch signalisiert, dass dessen Hände nur etwa 30s nach dem Auftragen des Desinfektionsmittels tatsächlich steril sind. Die Färbung besteht dann für einen bestimmten Zeitraum auf der Haut und bildet sich dann zurück. Eine solche Vorgehensweise bietet sich an, wenn besonders hohe Anforderungen an die Keimfreiheit gestellt werden. Den Nutzern wird hierdurch signalisiert, dass ein wirklich hoher Sterilitätsgrad der Hände nur eine sehr begrenzte Zeit vorliegt, sodass entsprechend nachdesinfiziert werden muss.

Vorteilhaft ist es, wenn die Zurückbildung der Färbung direkt mit der wieder einsetzenden Keimbesiedelung korreliert ist. Hierbei kann Zurückbildung der Färbung auch direkt durch die einsetzende neue Keimbesiedelung bewirkt werden, beispielsweise dann, wenn kontaminierte Gegenstände berührt worden sind. In einem solchen Fall ist es zweckmäßig, dass der Markierungsstoff mikrobiologisch abbaubar ist, wobei infolge des biologischen Abbaus, beispielsweise durch die Verarbeitung des Farbstoffs durch mikrobiologische Stoffwechselprozesse oder durch mikrobiologische Abbauprodukte, die markierende Wirkung nachlässt.

Der Markierungsstoff verbleibt in diesem Fall somit auch nach der Trocknung des Desinfektionsmittels sichtbar auf der Haut. Die Berührung eines Gegenstandes mit Keimbesiedelung führt aber nun dazu, dass die nun auf dem markierten Bereich vorhandenen Keime den Markierungsstoff zersetzen, indem diese ihn entweder assimilieren oder Enzyme freisetzen, die die Molekularstruktur des Markierungsstoffs zerstören.

Für verschiedene zeitliche und/oder räumliche Momente der Händedesinfektion, z.B. vor oder nach Betreten des Krankenzimmers, können auch mit unterschiedlichen Markierungsstoffen versehene Desinfektionsmittel verwendet werden. So entsteht eine weitere Signalisierung, bei der beispielsweise für jeden verbindlich signalisiert werden kann, dass insbesondere nach dem Betreten des Krankenzimmers blau eingefärbte Hände und vor dem Verlassen des Zimmers rot eingefärbte Hände erwünscht und gefordert sind.

Die Einfärbung kann auch auf eine bestimmte Desinfektionsmittelsorte hinweisen und eine Signalisierung der Stärke ihrer Wirksamkeit bewirken. Besonders stark wirksame Desinfektionsmittel färben hier z.B. die desinfizierten Bereiche pink ein, wobei hierdurch signalisiert wird, dass in diesem Falle ein besonders wirksames Desinfektionsmittel benutzt worden ist, bei dem eine längere Einwirkzeit vorliegt. Dies kann mit Hinweisen ergänzt werden, dass bestimmte Bereiche, beispielsweise bestimmte Behandlungsräume, nur dann betreten werden dürfen, wenn eine derartige Farbe auf der desinfizierten Haut nachweisbar ist. Hierzu kann insbesondere eine mit einem Zugangskontrollsystem gekoppelte Scaneinrichtung vorgesehen sein, die einen Zugang nur dann freischaltet, wenn der entsprechende Farbton registriert worden ist. Dies ist in Fig. 3 durch das Bezugszeichen 21 verdeutlicht. Zudem kann dadurch signalisiert werden, dass in Bereichen, in denen besondere Desinfektionsmittel mit besonderen Einfärbungen verwendet werden müssen, eine erhöhte Sorgfalt geboten ist.

Statt eines temporär sichtbaren Händedesinfektionsmittels, kann auch eine Ausführungsform des Desinfektionsmittels und dessen Verwendung vorgesehen sein die folgendermaßen funktionieren:
Nach dem Betreten eines Krankenzimmers wird zuerst ein Händedesinfektionsmittel mit einem Markierungsstoff benutzt, das die Hände zunächst dauerhaft einfärbt. Hierdurch wird innerhalb des Krankenzimmers signalisiert, dass die Hände desinfiziert und von allen von außen hereingetragenen Keimen gereinigt sind.

Vor dem Verlassen des Krankenzimmers wird dann ein zweites Händedesinfektionsmittel benutzt, dass eine entfärbende Substanz enthält, welche den von dem ersten Desinfektionsmittel aufgebrachten Farbstoff deaktiviert. Die Hände werden somit durch das zweite Desinfektionsmittel wieder entfärbt.

Als Beispiel für einen solchen Markierungsstoff in Verbindung mit einem entfärbenden Desinfektionsmittel können z.B. Betanin als Farbstoff und Natriumhypochlorid als entfärbendes Desinfektionsmittel genannt werden.

Eine solche Vorgehensweise ist dann sinnvoll, wenn beispielsweise eine Quarantäneeinrichtung betreten werden soll. Hierbei wird durch die Einfärbung der Hände zu Beginn signalisiert, dass durch die erste Desinfektion keine externen Keime eingeschleppt werden, während außerhalb des Quarantänebereichs durch die nun infolge der zweiten Desinfektion wieder entfärbten Hände signalisiert wird, dass keine Keime aus dem Quarantänebereich herausgeschleppt werden.

Die Umsetzung eines solchen Signalisierungsverfahrens kann durch die Entfärbung/Bleichung von Farbstoffen erfolgen, die durch die in Desinfektionsmitteln vorhandenen Oxidationsmittel wie Wasserstoffperoxid oder Natriumhypochlorid bewirkt werden. Als Beispiel können hierzu Lebensmittelfarbstoffe wie Anthocyane (Delphinidin, Cyanidin) oder Indigo, Methylenblau, Betanin und Brilliantblau verwendet werden.

Als Markierungsstoffe, die den Desinfektionsmitteln beigegeben werden können und die das oben erwähnte aktivierbare Markierungsverhalten zeigen, kommen folgende Farbstoffklassen in Frage.

Eine erste Farbstoffklasse wird durch photochrome Farbstoffe gebildet. Bei diesen erfolgt der markierende Farbwechsel durch Licht. Dieser unter Licht erfolgende Farbumschlag kann entweder dazu genutzt werden, um den Markierungsstoff zu aktivieren. Sobald dieser auf die Hand aufgetragen worden und dem Umgebungslicht ausgesetzt worden ist, zeigt sich die markierende Färbung. Diese kann ebenfalls durch das Umgebungslicht wieder ausgeblichen werden und verschwinden. Hierbei erweist es sich als zweckmäßig, dass der Zeitverlauf des Ausbleichens mit dem Zeitverlauf des zunehmenden Kontaminierungsvorgangs nach Abklingen der desinfizierenden Wirkung übereinstimmt, oder kürzer ist, sodass die ausgeblichene Farbe zuverlässig auf die fehlende Desinfektionswirkung hinweist.

Thermochrome Farbstoffe, die einen Farbwechsel bzw. eine Aktivierung durch Wärmeeinwirkung erfahren, können auf die Hauttemperatur reagieren. Möglich ist hier auch ein Zusammenwirken mit der durch die Verdunstung des Desinfektionsmittels ausgelösten Abkühlung der Hautoberfläche. Sobald das auf die Haut aufgetragene Desinfektionsmittel verdunstet, tritt erfahrungsgemäß die desinfizierende Wirkung ein. Damit ist eine Absenkung der Oberflächentemperatur der Haut verbunden. Dieser Temperaturumschlag kann genutzt werden, um den Farbumschlag des Markierungsstoffs zu bewirken und so den desinfizierten Zustand anzuzeigen.

Elektrochrome Farbstoffe können als Markierungsmittel eingesetzt werden, wobei hierzu die elektrochromen Eigenschaften des Farbstoffs bei dessen Aktivierung im Desinfektionsmittelspender ausgenutzt werden.

Solvatochrome Farbstoffe, deren Farbwechsel durch ein Lösungsmittel bewirkt wird, können als Markierungsstoff besonders vorteilhaft eingesetzt werden. Hier ist es insbesondere möglich, dass der Farbstoff bei der Mischung mit dem Desinfektionsmittel zunächst ausgebleicht wird, während er bei der Verdunstung des Desinfektionsmittels auf der Haut und der sich dadurch einstellenen desinfizierenden Wirkung zunächst wieder sichtbar wird. Günstig ist hier die Kopplung der solvatochromen Eigenschaft mit anderen Eigenschaften. So kann beispielsweise der solvatochrome Farbstoff auch ionochrom oder halochrom sein und auf pH-Änderungen reagieren, insbesondere zerfallen. Dadurch wird dessen Abbau auf der desinfizierten Haut bewirkt, die zu dessen Verblassen führt und somit das Nachlassen der desinfizierenden Wirkung und eine fortschreitende neue Kontamination sichtbar macht.

Auch ionochrome Farbstoffe, die einen Farbwechsel durch die Wechselwirkung mit Ionen zeigen, sind hier vorteilhaft einsetzbar. Bei deren Verwendung als Markierungsstoff zeigen diese beim Auftragen auf die Haut eine erste Farbe, beispielsweise blau, die den desinfizierten Zustand beispielsweise einer Hand signalisiert, während später unter dem Einfluss des von der Haut abgesonderten Schweißes und der darin befindlichen Ionen ein Farbumschlag zu einer anderen Farbe, beispielsweise zu Rot, erfolgt. Dies ist insbesondere dadurch bedeutsam, als das der desinfizierende Wirkstoff durch den Hautschweiß wieder abgewaschen wird und somit dessen Präsenz eng mit einer Neubesiedelung der Haut mit Keimen einher geht.

Auch tribochrome Farbstoffe, die einen Farbwechsel durch Reibung zeigen, oder piezochrome Farbstoffe, d.h. Farbstoffe, die einen Farbwechsel durch Druck zeigen, können als Markierungsstoff Anwendung finden. Hierbei ist insbesondere an mikroverkapselte Farbstoffe zu denken, die insbesondere in dem Desinfektionsmittelspender durch Reibung aktiviert werden können. Tribochrome und piezochrome Farbstoffe können aber auch anzeigen, ob eine Person das Desinfektionsmittel nur nachlässig über die Hand hat laufen lassen. In einem solchen Fall kann es beispielsweise so sein, dass hier die Hand überhaupt nicht oder nur mit einer warnenden Signalfärbung eingefärbt wird. Hingegen führt ein intensives Einmassieren des Desinfektionsmittels durch die dabei ablaufende Reibung auf der Haut und das Zusammendrücken der Handflächen zu einem Farbumschlag, der positiv signalisierend wird.

Darüber hinaus kommen zur Verwendung als Markierungsstoff aber auch alle Farbstoffe in Frage, die von Natur aus nicht ihre Farbigkeit ändern, aber unter bestimmten Eigenschaften zersetzt werden können und dadurch ihre Farbigkeit verlieren. Diese Eigenschaft ist für die oben genannten photochromen, thermochromen, elektrochromen, solvatochromen, ionochromen, tribochromen und piezochromen Farbstoffe ebenfalls erwünscht, um den oben erwähnten Farbumschlag nicht wieder rückgängig zu machen.

Die technische Umsetzung der Markierungsstoffe kann auch durch die Entfärbung/Bleichung von Farbstoffen durch die in Desinfektionsmitteln üblichen und zugelassenen Oxidationsmittel wie beispielsweise Wasserstoffperoxid oder Natriumhypochlorid erfolgen. Als Beispiel für derartige Markierungsstoffe sind hier Lebensmittelfarbstoffe wie Anthocyane, beispielsweise Delphinidin, Cyanidin, oder Indigo, Methylenblau, Betanin und Brilliantblau zu nennen.

Für das Rückbilden des Farbumschlags und der markierenden Wirkung des Markierungsstoffes können folgende Möglichkeiten genutzt werden:
Eine erste Möglichkeit besteht darin, einen Farbstoff mit einem möglichst niedrigen Siedepunkt auszuwählen. Der Farbstoff hat in diesem Fall beispielsweise einen Siedepunkt von etwa 30° und verflüchtigt sich durch die Temperaturänderung bei Hautkontakt. Hier ist zu beachten, dass sich der Farbstoff nicht zu schnell verflüchtigt, damit ein eigentlich noch desinfizierter Hautabschnitt bereits nicht wieder als kontaminiert ausgewiesen wird. Andererseits wird hierdurch ein Zuwachs an Sicherheit erreicht.

Eine weitere Möglichkeit besteht darin, dass sich der Farbstoff durch Lichteinstrahlung zersetzt. Hier ist darauf zu achten, dass die Zerfallszeit des Farbstoffs im Wesentlichen mit der Zeit einer verlässlichen Keimfreiheit des desinfizierten Hautareals übereinstimmt. Als weitere Möglichkeit bietet sich auch eine Lösung an, bei der der Farbstoff mit einem nanoverkapselten Lösungsmittel vermischt wird. Bei Luftkontakt brechen die Nanokapseln auf und das Lösungsmittel zersetzt den Farbstoff.

Möglich ist auch hier die Verwendung eines solvatochromen Farbstoffs, der durch bestimmte Lösungsmittel zersetzt wird und ausbleicht. Dies kann insbesondere ein Farbstoff sein, der dann zerfällt, wenn sich ein bestimmtes Hautmilieu mit einem Schweißfilm einstellt.

### Bezugszeichenliste

- 1: Desinfektionsmittelspender
- 2: Erster Vorratsbehälter
- 3: Desinfektionsmittel
- 4: Zweiter Vorratsbehälter
- 5: Markierungsstoff
- 6: Entnahmevorrichtung
- 7: Bügel
- 8: Ausgabeöffnung
- 9: Aktivierungsabschnitt
- 10: Desinfektionsmittelspender, Standardspender
- 11: Aktivierung durch Hautkontakt
- 12: Aktivierung durch Luftkontakt
- 13: Aktivierung durch Reibung
- 14: Aktivierung durch Lösemittel
- 15: Aktivierung durch elektromagnetische Strahlung
- 16: Aktivierung durch Elektrizität
- 17: Aktivierung durch Katalysator
- 18: Desinfektionsstation
- 19: Zugangskontrollsystem
- 20: Kamera
- 21: Scaneinrichtung an Raumzugang

## Patentansprüche

1. Nicht-therapeutisches und nicht-diagnostisches Verfahren zur öffentlichen Signalisierung eines Desinfektionszustandes von Personen mit folgenden Verfahrensschritten:
- Auftragen einer desinfizierenden Mischung bestehend aus einer desinfizierenden Komponente und einer Markierungskomponente auf ein zu desinfizierendes Hautareal an einer Desinfektionsstation (18) und/oder aus einem tragbaren Desinfektionsmittelspender,
- Einfärben des zu desinfizierenden Hautareals durch die Markierungskomponente während des Desinfektionsvorgangs,
- Sichtbare Persistenz der Einfärbung auf dem desinfizierten Hautareal, wobei der desinfizierte Zustand des Hautareals allgemein wahrnehmbar und detektierbar signalisiert wird,
- Abklingen der Persistenz der Einfärbung spätestens mit nachlassender Sterilität des Hautareals, wobei eine dadurch eintretende Ent- oder Umfärbung ebenfalls allgemein wahrnehmbar und detektierbar signalisiert wird, wobei
- die sichtbare Persistenz der Einfärbung auf dem desinfizierten Hautareal frühestens dann einsetzt, wenn durch die desinfizierende Komponente die Sterilität des Hautareals bewirkt wird,
**dadurch gekennzeichnet, dass**
das Abklingen der Persistenz der Markierkomponente durch das Verschwinden der Einfärbung infolge des Einwirkens der Umgebungsluft und/oder durch ein Ausbleichen infolge der Einwirkung von Umgebungslicht ausgelöst wird.

2. Verfahren nach Anspruch 1,
**dadurch gekennzeichnet, dass**
das desinfizierte und eingefärbte Hautareal durch eine Zugangskontrolleinrichtung (19) registriert wird, wobei im Ergebnis des Registrierungsvorgangs die Zugangskontrolleinrichtung einen Personenzutritt gewährt oder sperrt.

3. Verfahren nach Anspruch 1 oder 2,
**dadurch gekennzeichnet, dass**
das desinfizierte und eingefärbte Hautareal durch eine Bilderfassungs- und Bilderkennungseinrichtung (20, 21) registriert wird, wobei im Ergebnis einer ausgeführten Bilderfassung und Bilderkennung eine Desinfektionsüberwachung sich in einem Bereich aufhaltender Personenmengen und/oder durch vorbestimmte Durchgangsbereiche strömender Personenmengen ausgeführt wird.

4. Verfahren nach Anspruch 1,
**dadurch gekennzeichnet, dass**
die sichtbare Persistenz der Einfärbung auf dem Hautareal durch die abtötende Wirkung der desinfizierenden Komponente erzeugt wird, wobei dadurch entstehende biologische Abbauprodukte in der Markierkomponente eine sichtbare Farbreaktion erzeugen.

5. Verfahren nach Anspruch 1,
**dadurch gekennzeichnet, dass**
die sichtbare Persistenz der Einfärbung auf dem Hautareal durch das Einsetzen eines Verdunstungsvorgangs der desinfizierenden Komponente ausgelöst wird, wobei hierdurch in der Markierkomponente eine Farbreaktion erfolgt.

6. Verfahren nach Anspruch 1,
**dadurch gekennzeichnet, dass**
ein Abklingen der Persistenz der Markierkomponente durch ein Einwirken eines zweiten Desinfektionsmittels ausgelöst wird.

## Claims

1. A non-therapeutic and non-diagnostic method for public signalling of a disinfection state of persons, comprising the following method steps:
- applying a disinfecting mix consisting of a disinfecting component and a marking component to a skin area to be disinfected at a disinfection station (18) and/or from a portable disinfectant dispenser,
- colouring the skin area to be disinfected by the marking component during the disinfection process,
- visible persistence of the colouration on the disinfected skin area, wherein the disinfected state of the skin area is signalled in a generally perceivable and detectable manner,
- subsiding of the persistence of the colouration on or before subsiding sterility of the skin area, wherein a discolouration or change in colour caused thereby is also signalled in a generally perceivable and detectable manner, wherein
- the visible persistence of the colouration on the disinfected skin area commences on or after the sterility of the skin area is caused by the disinfecting component,
**characterised in that**
the subsiding of the persistence of the marking component is triggered by the disappearance of the colouration due to the impact of the ambient air and/or by a bleaching due to the impact of ambient light.

2. The method according to claim 1,
**characterised in that**
the disinfected and coloured skin area is registered by an access control arrangement (19), wherein, as a result of the registration process, the access control arrangement allows or blocks an access of persons.

3. The method according to claim 1 or 2,
**characterised in that**
the disinfected and coloured skin area is registered by an image capture and image recognition arrangement (20, 21), wherein, as a result of an image capture and image recognition that is performed, a disinfection monitoring of groups of persons located in an area and/or of groups of persons flowing through predetermined pass-through areas is performed.

4. The method according to claim 1,
**characterised in that**
the visible persistence of the colouration on the skin area is produced by the germicidal effect of the disinfecting component, wherein biological decomposition products generated thereby produce a visible colour reaction in the marking component.

5. The method according to claim 1,
**characterised in that**
the visible persistence of the colouration on the skin area is triggered by the commencement of an evaporation process of the disinfecting component, wherein a colour reaction thereby occurs in the marking component.

6. The method according to claim 1,
**characterised in that**
a subsiding of the persistence of the marking component is triggered by an impact of a second disinfectant.

## Revendications

1. Procédé non thérapeutique et non diagnostique de signalisation publique d'un état de désinfection de personnes, comprenant les étapes suivantes :
- application d'un mélange désinfectant, constitué d'un composant désinfectant et d'un composant de marquage, sur une zone cutanée à désinfecter au niveau d'une station de désinfection (18) et/ou à partir d'un distributeur portable de désinfectant,
- coloration de la zone cutanée à désinfecter par le composant de marquage pendant le processus de désinfection,
- persistance visible de la coloration sur la zone cutanée désinfectée, l'état désinfecté de la zone cutanée étant ainsi signalé de manière généralement perceptible et détectable,
- disparition progressive de la persistance de la coloration au plus tard lorsque la stérilité de la zone cutanée diminue, la décoloration ou le changement de couleur qui en résulte étant également signalé de manière généralement perceptible et détectable,
- la persistance visible de la coloration sur la zone cutanée désinfectée commençant au plus tôt lorsque la stérilité de la zone cutanée est obtenue par le composant désinfectant,
**caractérisé en ce que**
la disparition progressive de la persistance du composant de marquage est déclenchée par la disparition de la coloration sous l'effet de l'air ambiant et/ou par une décoloration sous l'effet de la lumière ambiante.

2. Procédé selon la revendication 1,
**caractérisé en ce que**
la zone cutanée désinfectée et colorée est enregistrée par un dispositif de contrôle d'accès (19), le dispositif de contrôle d'accès autorisant ou refusant l'accès d'une personne à l'issue de l'opération d'enregistrement.

3. Procédé selon la revendication 1 ou 2,
**caractérisé en ce que**
la zone cutanée désinfectée et colorée est enregistrée par un dispositif de capture et de reconnaissance d'images (20, 21), et, à l'issue d'une opération effectuée de capture et de reconnaissance d'images, une surveillance de la désinfection est alors effectuée parmi des foules de personnes séjournant dans une zone et/ou parmi des foules de personnes traversant des zones de passage prédéterminées.

4. Procédé selon la revendication 1,
**caractérisé en ce que**
la persistance visible de la coloration sur la zone cutanée est générée par l'effet bactéricide du composant désinfectant, les produits de dégradation biologique ainsi formés dans le composant de marquage provoquant une réaction colorée visible.

5. Procédé selon la revendication 1,
**caractérisé en ce que**
la persistance visible de la coloration sur la zone cutanée est déclenchée par le début d'un processus d'évaporation du composant désinfectant, ce qui provoque une réaction colorée dans le composant de marquage.

6. Procédé selon la revendication 1,
**caractérisé en ce que**
la disparition progressive de la coloration du composant de marquage est déclenchée par l'action d'un deuxième désinfectant.
